# EUROPEAN PATENT SPECIFICATION

(11) **EP 0 751 755 B1**
(45) Date of publication and mention of the grant of the patent: **18.07.2001**
(21) Application number: 95912500.6
(22) Date of filing: 20.03.1995
(51) Int. Cl.: A61F 6/22

(54) **BLOCKING UNIT FOR A FALLOPIAN TUBE**
BLOCKIEREINRICHTUNG FÜR EINEN EILEITER
UNITE D'OBTURATION D'UNE TROMPE UTERINE

(30) Priority: 18.03.1994 NL 9400436
(43) Date of publication of application: 08.01.1997
(73) Proprietor: Doorschodt, Benedict Marie, 1091 GB Amsterdam (NL)
(72) Inventor: Doorschodt, Benedict Marie, 1091 GB Amsterdam (NL)
(74) Representative: de Bruijn, Leendert C.
(86) International application number: NL9500102
(87) International publication number: WO9525490

(56) References cited:
- EP-A- 0 010 812
- EP-A- 0 565 395
- WO-A-93/06781
- DE-A- 2 404 605
- US-A- 4 595 000

## Description

The invention relates to a blocking unit according to the preamble of Claim 1.

A blocking device of this type ,is known from US-A-A.4,595,000.

A problem when inserting such contraceptive devices is, on the one hand, that there must be sufficient safeguard that the device will be held in place in the Fallopian tube in all circumstances and, on the other hand, that the insertion is as simple as possible.

In the case of the device according to the above US patent the insertion is facilitated by using an hysteroscope having a channel for guiding a unit. After insertion the hysteroscope is removed from the human body by retracting. Because of its removable nature there are restrictions with regard to the shape of the blocking units.

German Offenlegungsschrift 2,404,605 discloses a blocking device which consists of an elongated element whose free ends are each inserted into a Fallopian tube. This eliminates a number of problems as regards holding the device in place in the Fallopian tube, because when the blocking device is coming out of one Fallopian tube it will have to go further into the other Fallopian tube, which is counteracted by said other tube. However, it has been found that such a design does not give sufficient guarantee to permit the end to be made without a widened part. For that reason, this German Offenlegungsschrift 2,404,605 proposes making the ends widened, with all the above-described consequences which this entails with regard to fitting and positioning.

The object of the present invention is to provide a blocking unit which does not have the abovementioned disadvantages.

This object is achieved in the case of a blocking unit of the type described above by the characterizing measures of Claim 1.

Providing the retaining means ensures that the free, widened end can have relatively small dimensions while it is being inserted into the Fallopian tube because of the presence of the retaining means. If the retaining means are then taken away in some way which is known in the prior art, the free end can widen, for example through its own resilience, and become fixed in the Fallopian tube.

Consequently, on the one hand, it is not necessary to administer an anaesthetic while, on the other hand, it is possible to widen the end further than according to the prior art, with the result that a more positive positioning of the free end is produced through engagement with the Fallopian tube.

As already stated above, the retaining means can comprise all means known in the prior art, which can be operated externally by, for example, pulling on cords and other constructions.

The retaining means comprise a bioabsorbable or biodegradable material, having such composition that they dissolve in body fluids, and more particularly the fluids near the Fallopian tube, within a few hours of insertion.

On the one hand, this means that a particularly simple construction will suffice while, on the other, there is total certainty that no harmful effect whatsoever can occur for the user.

The widening of the free end and the release by the retaining means can be achieved by all means known in the prior art. A particularly simple embodiment comprises making the free end from a permanently elastic, foldable material. The free end is inserted, held by the retaining means in the folded position, into the Fallopian tube, and after the retaining means cease to be effective the material can unfold elastically.

The blocking unit can also comprise an applicator inside which the blocking device is disposed in the folded state. If said applicator is then placed in the Fallopian tube with the blocking device fitted therein, and the blocking device is subsequently released by moving the applicator out of the Fallopian tube, leaving the blocking device in place, optimum positioning can be provided.

The element to which the free end is attached can be of any type known in the prior art, but is preferably straight. In this case the element is slightly elastic, so that it adapts to the shape of the user's body.

The retaining means preferably comprise cap-shaped parts. For easy insertion, these parts are provided with a round tip, so that provision is made for simple insertion into the Fallopian tube. It is endeavoured to make the free end relatively flexible and the part which faces more towards the uterus stiffer. For this purpose, the free end is made of a relatively thin material, while the part of the blocking device facing the uterus is made of a thicker material. This produces a construction which, on the one hand, is sufficiently stiff to remain in position and, on the other hand, causes no damage to the Fallopian tube.

Optimum positioning of the blocking device can be obtained by making it double. Such a design consists of two blocking devices of the type described above, interconnected in line with each other. In the case of such a construction the various parts are preferably of hollow design, so that an open connection between the two Fallopian tubes exists, on the one hand, but there is no connection between the Fallopian tubes and the uterus, on the other. Consequently, pressure variations arising through an egg being released are absorbed by the opposite Fallopian tube.

In order to facilitate the insertion and monitoring of the correct position of such a double design, the central part is provided with indicator means between the two blocking devices. In this way the person placing the double construction can feel whether the central part is actually lying in the centre between the two Fallopian tubes.

These indicator means preferably comprise a thickened part. Such a thickened part will likewise be gripped by the adjacent tissue material, with the result that further positioning of the blocking device is provided.

However, it is also possible to design the blocking device individually for each Fallopian tube. In this case said blocking device, in addition to the widened free end, preferably consists of a free end which connects thereto and is widened in the other direction, and which should be situated in the uterine cavity. At least one of these widened free ends is preferably funnel-shaped.

An essentially cylindrical part can be present between the widened ends, which part serves for fixing in the Fallopian tube.

The invention will be explained in greater detail below with reference to exemplary embodiments shown in the drawing, in which:
Fig. 1 shows a first embodiment of the device according to the invention, shown in cross-section, fitted in the Fallopian tubes;
Fig. 2 shows a perspective view of the device according to Fig. 1, in which the retaining means to be used here are shown separated therefrom;
Fig. 3 shows the device according to Fig. 2, with the retaining means fitted;
Fig. 4 shows in perspective a further embodiment of the device according to the invention;
Fig. 5 shows a further variant of the device according to Fig. 4; and
Fig. 6 shows diagrammatically a device for inserting the device according to Fig. 5.

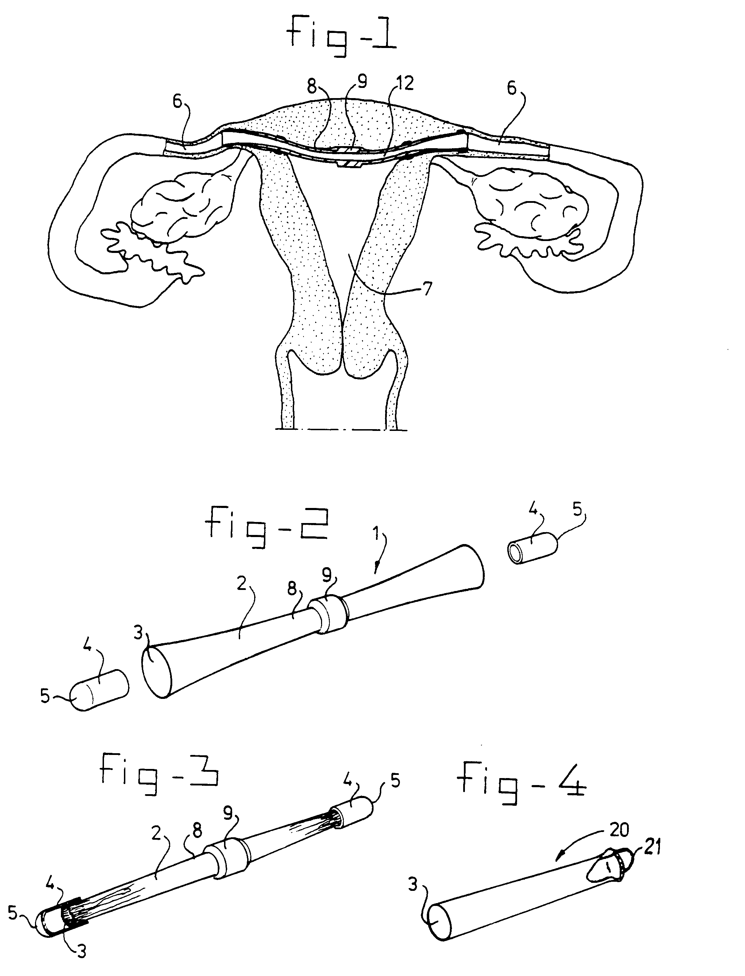

Figures 4 to 6 do not show retaining means comprising a bioabsorbable material as claimed in claim 1.

In the figures a first double embodiment of the blocking device according to the invention is indicated in its entirety by 1. It consists of opposite elongated parts 2, which are connected by a central part 8 in which a thickened part is disposed. Free ends 3 connect to the elongated parts 2. The double embodiment is provided with a passage 12 extending over the full length. The free ends 3 have a substantially larger radius than the elongated part 2 and central part 8. The transition from this region with larger radius to the central part is conical. As can be seen from Fig. 1, the wall thickness of the free ends 3 is considerably smaller than that of the elongated part 2 or the central part 8.

The positioning of the blocking device 1 according to the invention relative to the Fallopian tubes 6 and uterus 7 can also be seen from Fig. 1. The free end 3, which is relatively wide and is made of thin flexible material, provides a seal between the device and the Fallopian tube. The elongated part 2 and the central part 8 provide fixing. Moreover, a further seal is provided through the isthmus gripping the elongated part, giving additional security in this way. Correct positioning can be checked by feeling for the presence of thickened part 9.

Fig. 2 also shows caps 4, provided with a rounded end 5. Said caps are made of bioabsorbable material, i.e. a material which can be readily absorbed by the body fluids at the position of the uterus or Fallopian tube. The caps 4 have external dimensions corresponding approximately to the diameter of the central part 8. Inside the caps 4 there is a receptacle for accommodating the folded free ends 3. This is shown in Fig. 3. For this purpose, it is, of course, necessary that at least the free ends 3 are made of a readily folding, springy, elastic material.

In general, the blocking device will be made of a plastic which behaves neutrally with respect to the body and has the above-described properties permanently.

The above-described device functions as follows:

The blocking device is supplied in the state shown in Fig. 3. A gynaecologist or other expert then inserts the blocking device into the uterus 7 of the user and into the Fallopian tubes 6. The correct positioning can be checked by, inter alia, checking the position of thickened part 9. Insertion of the free ends 3 into the Fallopian tubes 6 is relatively simple, due to the small external diameter of caps 4. It is not necessary to administer an anaesthetic in this case, because this operation causes little pain to the user.

Immediately after the insertion, the bioabsorbable material of caps 4 is dissolved, so that the free ends unfold and provide a locking relative to the Fallopian tube or wall of the uterus. The bioabsorbable material is removed in a harmless manner, known in the prior art. Due to the soft nature of the end 3, the device can be removed easily if necessary, without causing damage to the tube.

Fig. 4 shows a variant of an embodiment, in which a "half" blocking device is being used, i.e. a single blocking device, which is indicated by 20. It has been found that, with the combination of cap 4 and the outward springing free end, the fitting is so simple and locking in the Fallopian tube is so good that the added fixing of the position of the blocking device by interconnecting two such blocking devices is not always necessary. Loop 21 is present for removal of the device.

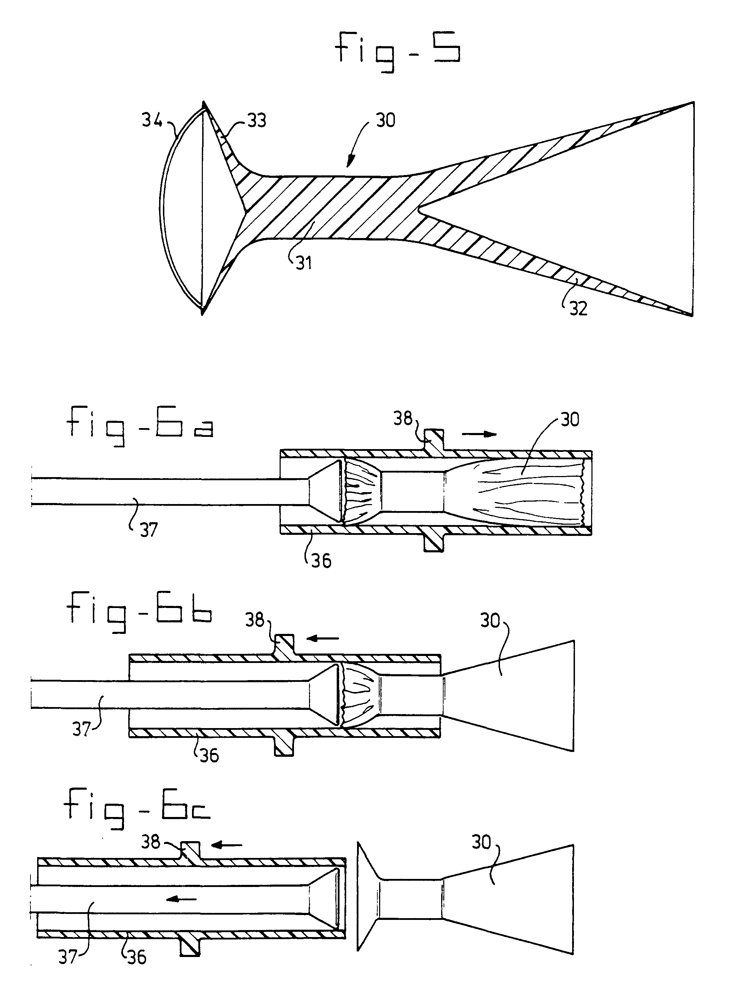

Fig. 5 shows a further embodiment of the device according to the invention.

Like the embodiment shown in Fig. 4, this is a single blocking device. It is indicated in its entirety by 30 and comprises an essentially cylindrical central part 31, a flared part 32 which is intended for placing in the Fallopian tube, and a flared part 33 which should be situated in the uterine cavity after insertion. Engagement means 34 are fitted on said flared part 33. They are used for the subsequent removal of the device if, for example, fertility has to be restored.

The central part in this embodiment is of such cylindrical design that the sphincter muscle present in the Fallopian tube grips this smooth part 31 and fixes the device in this way.

Further adhesion can be provided by making part 31 thinner, winding a thread around it and placing hydrogel on it, which causes intergrowth with the surrounding tissue.

For detection of the device, for example by X-ray or ultra-sound scan, certain parts of the device shown in Fig. 5 can be of special design. For example, the ends can be provided with a ring which reacts to said detection means.

It is also possible to fit a spring or the like which facilitates the removal in a funnel shape of both parts 32 and 33. Such a spring can comprise a memory metal, which becomes active at the temperature of the human body, in order to facilitate removal in a funnel shape. This is important in particular if the device is made of a weak material, such as a silicone material.

Fig. 6 shows diagrammatically an aid for fitting the device shown in Fig. 5. It consists of an applicator 36 which is provided with a plunger 37 which is movable relative thereto. The applicator is provided with a stop 38. Device 30 is supplied folded in this applicator.

The doctor then inserts the applicator into the Fallopian tube as far as the stop 38, so that a certain indication is obtained that the applicator 36 has been inserted to sufficient depth. Thereafter, plunger 37 is held in place and applicator 36 is moved back, as shown diagrammatically in Fig. 6b. The end position is shown in Fig. 6c, in the case of which the device is free.

It can be seen from Fig. 6 that the device is inserted from the uterus side.

However, it is also possible to insert the device according to the invention operatively from the side of the Fallopian tube through the abdominal cavity. In such a case the device 30 has to be fitted in applicator 36 exactly the other way round.

Although the invention has been described above with reference to preferred embodiments, it must be understood that numerous modifications can be made thereto without going beyond the scope of the present application, as described in the appended claims. For example, the retaining means can comprise means which react to temperature or moisture.

It is also possible to provide the device with means which influence fertility. For example, copper or a hormone-releasing material may be present.

## Claims

1. Blocking unit, comprising retaining means and a blocking device (20) for a Fallopian tube, having an elongated part (2) with at least a widened free end (3), which end must be placed in the Fallopian tube (6), wherein during the fitting said retaining means are present at the end (3), which retaining means limit the external dimensions of the free end of the elongated part and are fitted in such a way that they are removable, characterised in that the retaining means comprise a bioabsorbable material.

2. Blocking unit according to Claim 1, in which at least the free end (3) is made of a permanently elastic, foldable material.

3. Blocking unit according to one of the preceding claims, in which the elongated part is provided with a second widened free end (33), which lies opposite the first widened free end (32) and must be placed in the working position in the uterus.

4. Blocking unit according to Claim 3, in which an essentially cylindrical part (31) is provided between the widened ends (32,33).

5. Blocking unit according to one of the preceding claims, in which an element to which the free end is attached is essentially straight before its insertion.

6. Blocking unit according to one of the preceding claims, in which the retaining means are cap-shaped means (4).

7. Blocking unit according to one of the preceding claims in which the free end is made of a relatively thin material, and the part of the device which is to face the uterus is made of a relatively thick material.

8. Blocking unit according to one of the preceding claims, in conjunction with Claim 3, in which the blocking unit comprise an applicator (36) for accommodating the blocking device (20).

9. Blocking unit according to Claim 8, in which the applicator (36) is provided with stop means (38).

10. Blocking arrangement, comprising two blocking units according to one of Claims 1 - 9, disposed essentially in line with each other, and connected by a central part (8).

## Patentansprüche

1. Blockiereinheit, die eine Halteeinrichtung und eine Blockiervorrichtung (20) für einen Eileiter umfasst und einen länglichen Teil (2) mit wenigstens einem aufgeweiteten freien Ende (3) aufweist, wobei das Ende in dem Eileiter (6) angeordnet werden muss und wobei beim Einpassen die Halteeinrichtung an dem Ende (3) vorhanden ist und die Halteeinrichtung die Außenabmessungen des freien Endes des länglichen Teils beschränkt und so angebracht ist, dass sie entfernt werden kann, **dadurch gekennzeichnet,** dass die Halteeinrichtung ein biologisch absorbierbares Material umfasst.

2. Blockiereinheit nach Anspruch 1, wobei wenigstens das freie Ende (3) aus einem dauerhaft elastischen, zusammendrückbaren Material besteht.

3. Blockiereinheit nach einem der vorangehenden Ansprüche, wobei der längliche Teil mit einem zweiten aufgeweiteten freien Ende (33) versehen ist, das dem ersten aufgeweiteten freien Ende (32) gegenüberliegt und in der Funktionsposition im Uterus angeordnet sein muss.

4. Blockiereinheit nach Anspruch 3, wobei ein im wesentlicher zylindrischer Teil (31) zwischen den aufgeweiteten Enden (32, 33) vorhanden ist.

5. Blockiereinheit nach einem der vorangehenden Ansprüche, wobei ein Element, an dem das freie Ende angebracht ist, vor dem Einführen im Wesentlichen gerade ist.

6. Blockiereinheit nach einem der vorangehenden Ansprüche, wobei die Halteeinrichtung eine kappenförmige Einrichtung (4) ist.

7. Blockiereinheit nach einem der vorangehenden Ansprüche, wobei das freie Ende aus einem relativ dünnen Material besteht und der Teil der Vorrichtung, der dem Uterus zugewendet sein soll, aus einem relativ dicken Material besteht.

8. Blockiereinheit nach einem der vorangehenden Ansprüche in Verbindung mit Anspruch 3, wobei die Blockiereinheit einen Applikator (36) umfasst, der die Blockiervorrichtung (20) aufnimmt.

9. Blockiereinheit nach Anspruch 8, wobei der Applikator (36) mit einer Anschlageinrichtung (38) versehen ist.

10. Blockieranordnung, die zwei Blockiereinheiten nach einem der Ansprüche 1-9 umfasst, die im Wesentlichen hintereinander angeordnet und durch ein Mittelteil (8) miteinander verbunden sind.

## Revendications

1. Unité d'obturation comprenant des moyens de retenue et un dispositif d'obturation (20) pour une trompe de Fallope, possédant une partie allongée (2) avec au moins une extrémité libre élargie (3), ladite extrémité devant être installée dans la trompe de Fallope (6), dans laquelle au cours de la mise en place lesdits moyens de retenue sont présents à l'extrémité (3), lesdits moyens de retenue limitant les dimensions externes de l'extrémité libre de la partie allongée et étant installés d'une manière telle qu'ils puissent être enlevés, caractérisée en ce que les moyens de retenue comprennent un matériau bioabsorbable.

2. Unité d'obturation selon la revendication 1, dans laquelle l'extrémité libre (3) au moins est faite dans un matériau pliable élastique de manière permanente.

3. Unité d'obturation selon l'une des revendications précédentes, dans laquelle la partie allongée est munie d'une seconde extrémité libre élargie (33), qui s'étend à l'opposé de la première extrémité libre élargie (32) et devant être placée dans la position de travail dans l'utérus.

4. Unité d'obturation selon la revendication 3, dans laquelle une partie essentiellement cylindrique (31) est ménagée entre les extrémités élargies (32, 33).

5. Unité d'obturation selon l'une des revendications précédentes, dans laquelle un élément, auquel l'extrémité libre est attachée, est essentiellement droit avant son insertion.

6. Unité d'obturation selon l'une des revendications précédentes, dans laquelle les moyens de retenue sont des moyens en forme de bouchon (4).

7. Unité d'obturation selon l'une des revendications précédentes, dans laquelle l'extrémité libre est faite dans un matériau relativement fin, et la partie du dispositif devant faire face à l'utérus est faite d'un matériau relativement épais.

8. Unité d'obturation selon l'une des revendications précédentes en combinaison avec la revendication 3, dans laquelle l'unité d'obturation comprend un applicateur (36) pour installer le dispositif d'obturation (20).

9. Unité d'obturation selon la revendication 8, dans laquelle l'applicateur (36) est pourvu de moyens de butée (38).

10. Ensemble d'obturation, comprenant deux unités d'obturation selon l'une des revendications 1-9, disposées essentiellement en ligne l'une par rapport à l'autre, et reliées par une partie centrale (8).
